Europäisches Patentamt

European Patent Office

Office européen des brevets

(19)

(11) EP 0 634 388 B1

(12) **EUROPÄISCHE PATENTSCHRIFT**

(45) Veröffentlichungstag und Bekanntmachung des Hinweises auf die Patenterteilung:
04.06.1997 Patentblatt 1997/23

(51) Int Cl.$^6$: **C07C 68/00**, C07C 69/96

(21) Anmeldenummer: 94110394.7

(22) Anmeldetag: 04.07.1994

(54) **Verfahren zur Herstellung von Dialkylcarbonaten**

Process for the preparation of dialkyle carbonates

Procédé de préparation de carbonates de dialkyle

(84) Benannte Vertragsstaaten:
BE DE ES FR GB IT NL

(30) Priorität: 15.07.1993 DE 4323689

(43) Veröffentlichungstag der Anmeldung:
18.01.1995 Patentblatt 1995/03

(73) Patentinhaber: BAYER AG
51368 Leverkusen (DE)

(72) Erfinder:
• Hable, Konrad
 D-51375 Leverkusen (DE)

• Klausener, Alexander, Dr.
 D-50670 Köln (DE)
• Kricsfalussy, Zoltan, Dr.
 D-51375 Leverkusen (DE)
• Landscheidt, Heinz, Dr.
 D-47057 Duisburg (DE)
• Wolters, Erich, Dr.
 D-50939 Köln (DE)
• Zirngiebl, Eberhard, Dr.
 D-51061 Köln (DE)

(56) Entgegenhaltungen:
EP-A- 0 523 508

**Beschreibung**

Die vorliegende Erfindung betrifft ein Verfahren zur Herstellung von Dialkylcarbonaten durch Umsetzung von Kohlenmonoxid (CO) mit Alkylnitriten in Gegenwart eines heterogenen Platinmetall-Katalysators, wobei die Aktivität des Katalysators durch Zugabe geringer Mengen Halogen aufrechterhalten wird.

Dialkylcarbonate sind von allgemeiner chemischer und technischer Bedeutung. So ist beispielsweise Diethylcarbonat ein ausgezeichnetes Lösungsmittel im mittleren Siedebereich. Des weiteren sind die Dialkylcarbonate ausgezeichnete Carbonylierungs- und Acylierungsreagenzien. Sie haben große Bedeutung bei der Herstellung von anderen Carbonaten, von Urethanen und von Harnstoffen. Schließlich eignen sie sich aufgrund ihres hohen Sauerstoffgehalts als Treibstoffadditive zur Verbesserung der Klopffestigkeit von Ottokraffstoffen.

Es ist bekannt, Dialkylcarbonate durch Umsetzungen von Phosgen bzw. von Chlorameisensäurealkylestern mit Alkoholen herzustellen.

Es besteht jedoch ein steigendes Interesse daran, den Einsatz des giftigen Phosgens bzw. der davon abgeleiteten Zwischenprodukte wie Chlorameisensäureester durch andere Verfahren abzulösen.

Hier sind insbesondere Verfahren wichtig, bei denen CO in der Gasphase mit Alkylnitriten an heterogenen Platinmetall-Katalysatoren umgesetzt wird.

So wird in der Zeitschrift für Katalytische Forschung (China) Vol.10(1) S.75-78 (März 1989) die Umsetzung von CO und Methylnitrit an einem $PdCl_2$-haltigen Aktivkohle-Katalysator beschrieben, wobei neben Dimethyloxalat größtenteils Dimethylcarbonat entsteht.

In DE-OS 41 23 603 wird durch Anwendung eines heterogenen Palladiumchlorid-Katalysators mit $\gamma$-$Al_2O_3$ als Trägermaterial eine hohe Selektivität, sowohl bezogen auf CO als auch auf Methylnitrit, bei gleichzeitig hohem Umsatz erzielt. Allerdings muß dem Eduktgemisch zur Aufrechterhaltung der katalytischen Aktivität Chlorwasserstoffgas in Mengen von bis zu 1000 ppm (Volumen) zugesetzt werden. Dies kann, wenn man an die Ausübung dieses Verfahrens in technischem Maßstab denkt, zu Korrosionsproblemen führen.

Überraschenderweise konnten die dargestellten Nachteile durch Zugabe geringer Mengen elementaren Halogens, insbesondere durch Zugabe von Chlor oder Brom, zum Reaktionsgemisch überwunden werden.

Es wurde ein Verfahren zur Herstellung von Dialkylcarbonaten der allgemeinen Formel

$$O=C(OR)_2 \qquad (I),$$

worin

R für geradkettiges oder verzweigtes $C_1$-$C_4$-Alkyl

steht,

durch Umsetzung von Kohlenmonoxid (CO) mit Alkylnitriten der Formel

$$RONO \qquad (II),$$

worin

R die angegebene Bedeutung hat,

in Anwesenheit eines Inertgases sowie in Anwesenheit des zugrundeliegenden Alkohols ROH sowie in Anwesenheit oder Abwesenheit von NO an einem heterogenen Platinmetall-Trägerkatalysator, bei erhöhter Temperatur in kontinuierlicher Gasphasenreaktion gefunden, das dadurch gekennzeichnet ist, daß bei einem Volumenverhältnis von Alkylnitrit:CO = 0,1 bis 10:1, bevorzugt 0,2 bis 4:1, besonders bevorzugt 0,3 bis 3:1, einem Druck von 0,5 bis 10 bar, bevorzugt 0,8 bis 7 bar, besonders bevorzugt 1 bis 6 bar, ganz besonders bevorzugt 1 bis 5 bar, und einer Temperatur von 50 bis 170°C, bevorzugt 70 bis 160°C, besonders bevorzugt 70 bis 150°C, gearbeitet wird, wobei absatzweise oder kontinuierlich Halogen zugesetzt wird.

Für das ertindungsgemäße Verfahren eignen sich alle dem Fachmann bekannten Platinmetall-Trägerkatalysatoren, bevorzugt Palladium-Trägerkatalysatoren, besonders bevorzugt Palladium(II)-Halogenid-Trägerkatalysatoren, insbesondere bevorzugt $PdCl_2$-haltige Trägerkatalysatoren auf Trägern wie Aluminiumoxid, Spinelle, Silikate, Montmorillonite, Zeolithe, Aktivkohlen, Molekularsiebe, Diatomeenerden, Siliciumcarbid, Siliciumdioxid, Metalloxide, Metallphosphate, Heteropolysäuren und andere.

Die dem erfindinngsgemäßen Verfahren zugrundliegende Umsetzung erfolgt im Sinne der nachfolgenden Reaktionsgleichung:

$$CO + 2\ RONO \rightarrow O=C(OR)_2 + 2\ NO$$

R ist hierbei geradkettiges oder verzweigtes Alkyl mit 1 - 4 C-Atomen, beispielsweise Methyl, Ethyl, Propyl, Isopropyl, Butyl, Isobutyl, in bevorzugter Weise Methyl und Ethyl und in besonders bevorzugter Weise Methyl.

Während es grundsätzlich möglich ist, CO mit einem Alkylnitrit ohne weitere Gasgemischkomponente umzusetzen, beispielsweise wenn man sich in der Gemischzusammensetzung außerhalb der Explosionsgrenzen befindet, wird vielfach ein Inertgas zur Verdünnung der Reaktionspartner herangezogen. Solchermaßen verwendbare Inertgase sind beispielsweise Edelgase, Stickstoff und Kohlendioxid, bevorzugt Argon, Stickstoff oder Kohlendioxid, besonders bevorzugt Stickstoff und Kohlendioxid.

Die Menge des Inertgases beträgt 20 bis 80 Vol.-%,

bezogen auf das gesamte in den Reaktor einströmende Gasvolumen. Das Inertgas, gegebenenfalls darin enthaltene nicht umgesetzte Restmengen der Reaktionspartner sowie andere gasförmige Reaktionshilfsstoffe oder Nebenprodukte können im Sinne eines kontinuierlich arbeitenden Kreisprozesses, wie er beispielsweise, wenngleich auch in technisch nicht völlig zufriedenstellender Form in der Patentanmeldung EP 523 728 beschrieben wird, recycliert werden, wobei jedoch gegebenenfalls kontinuierlich oder absatzweise bestimmte Teilvolumina des Kreisgasgemisches ausgeschleust werden.

Das Volumenverhältnis der zur Umsetzung gebrachten Reaktionspartner Alkylnitrit und CO zueinander beträgt 0,1 bis 10:1, bevorzugt 0,2 bis 4:1, besonders bevorzugt 0,3 bis 3:1.

Das umzusetzende Gasgemisch kann weiterhin geringe Mengen an Alkohol ROH, beispielsweise in einer Menge von 0 bis 10 Vol.-% und geringe Mengen an NO, beispielsweise in einer Menge von 0 bis 10 Vol.-%, beides bezogen auf das Gesamtvolumen des einzusetzenden Gasgemisches, enthalten. Solche Zusätze an ROH bzw. NO können etwa aus der Herstellung des Alkylnitrits stammen und beispielsweise mit diesem in das Reaktionsgasgemisch hineingebracht werden.

Halogen im Sinne der Erfindung ist Fluor, Chlor, Brom und Iod, bevorzugt Chlor und Brom , besonders bevorzugt Chlor.

Das Halogen kann als solches dem Reaktionsgemisch gasförmig hinzudosiert werden, und zwar in reiner Form oder im Gemisch mit anderen Gasen, bevorzugt im Gemisch mit einem Inertgas, besonders bevorzugt im Gemisch mit Stickstoff oder Kohlendioxid. Es kann jedoch dem Reaktionsgemisch auch in gelöster Form hinzudosiert werden, wobei als Lösungsmittel vorzugsweise einer der im Reaktionsgemisch vorhandenen Stoffe, beispielsweise der dem Alkylnitrit zugrundeliegende Alkohol, dienen kann.

Dabei kann die Konzentration des Halogens im Gasstrom 1 bis 1000 ppm (Volumen), bevorzugt 10 bis 500 ppm (Volumen) betragen.

## Beispiel

## Definitionen

Die Raumzeitausbeute (Space Time Yield, STY) in [g/l∗h] für Dimethylcarbonat in den Beispielen berechnet sich nach

$$STY = \frac{m_{DMC}}{V_{Kat} * t}$$

wobei $m_{DMC}$ die Menge des gebildeten Dimethylcarbonats (DMC), $V_{Kat}$ das Volumen der Katalysatorschüttung und t die Zeit bedeuten.

Die Selektivität S [%] wird berechnet nach

$$S = \frac{n_{DMC}}{n_{DMC} + 2 * n_{ODME} + n_{AME} + n_{FDA}} * 100 [\%]$$

wobei

$n_{DMC}$ = Stoffmenge Dimethylcarbonat
$n_{ODME}$ = Stoffmenge Oxalsäuredimethylester
$n_{AME}$ = Stoffmenge Ameisensäuremethylester
$n_{FDA}$ = Stoffmenge Formaldehyddimethylacetal

bedeuten.

## Katalysatorherstellung

100 ml Aluminiumoxid-Pellets wurden mit einer wäßrigen $Li_2PdCl_4$-Lösung getränkt und das Produkt bei 80°C im Vakuum getrocknet.

Der Katalysator enthielt dann 8 g Pd/l.

## Verfahrensbeschreibung

In einen vertikal aufgestellten Rohrreaktor (Glas, Länge 50 cm, Durchmesser 4 cm) wurden zwischen eine Füllung aus Raschig-Ringen 20 ml des beschriebenen Katalysators eingebracht.

Das Glasrohr wurde auf 90°C erhitzt, und ein Gasgemisch aus 55 Vol.-% $N_2$, 20 Vol.-% Methylnitrit, 20 Vol.-% CO und 5 Vol.-% Methanol wurde hindurchgeleitet, wobei diesem Gasgemisch 100 ppm $Cl_2$ (Volumen) zugesetzt wurden.

Das dem Reaktor entströmende Gas wurde auf 5°C gekühlt und die erhaltene kondensierte Phase wurde mittels Gaschromatographie untersucht.

Die nicht kondensierten Produkte wurden mittels IR-Spektroskopie und Massenspektroskopie erfaßt.

Dimethylcarbonat wurde nach 2 h mit einer Raumzeitausbeute von STY = 190 g/∗h und einer Selektivität von S = 99 % gebildet.

Auch nach 60 h Reaktionszeit betrugen die Raumzeitausbeute STY = 190 gl/∗h und die Selektivität S = 99 %.

## Patentansprüche

**1.** Verfahren zur Herstellung von Dialkycarbonaten der Formel

$$O = C(OR)_2,$$

worin

R für geradkettiges oder verzweigtes $C_1$-$C_4$-Alkyl, bevorzugt Methyl oder Ethyl, besonders bevorzugt Methyl, steht,

durch Umsetzung von Kohlenmonoxid (CO) mit Alkylnitriten der Formel

RONO,

worin

R die angegebene Bedeutung hat,

in Anwesenheit eines Inertgases sowie in Anwesenheit des zugrundeliegenden Alkohols sowie in Anwesenheit oder Abwesenheit von NO an einem Platinmetall-Trägerkatalysator, bevorzugt an einem Palladium-Trägerkatalysator, besonders bevorzugt an einem Pd(II)-Halogenid-Trägerkatalysator, insbesondere bevorzugt an einem $PdCl_2$-haltigen Trägerkatalysator, bei erhöhter Temperatur in kontinuierlicher Gasphasenreaktion,
dadurch gekennzeichnet, daß bei einem Volumenverhältnis von Alkylnitrit:CO = 0,1 bis 10:1, bevorzugt 0,2 bis 4:1, besonders bevorzugt 0,3 bis 3:1, einem Druck von 0,5 bis 10 bar, bevorzugt 0,8 bis 7 bar, besonders bevorzugt 1 bis 6 bar, ganz besonders bevorzugt 1 bis 5 bar, und einer Temperatur von 50 bis 170°C, bevorzugt 70 bis 160°C, besonders bevorzugt 70 bis 150°C gearbeitet wird, wobei absatzweise oder kontinuierlich Halogen zugesetzt wird.

2. Verfahren nach Anspruch 1, dadurch gekennzeichnet, daß Halogen in einer solchen Menge zugesetzt wird, daß das Eduktgas das Halogen in einer Konzentration von 1 bis 1000 ppm (Volumen), bevorzugt 10 - 500 ppm (Volumen) enthält.

3. Verfahren nach Anspruch 1, dadurch gekennzeichnet, daß Chlor oder Brom, bevorzugt Chlor zugesetzt wird.

## Claims

1. Process for the preparation of dialkyl carbonates of the formula

$$O=C\,(OR)_2,$$

wherein

R represents linear or branched $C_1$-$C_4$-alkyl, preferably methyl or ethyl and particularly preferably methyl,

by reacting carbon monoxide (CO) with alkyl nitrites of the formula

RONO,

wherein

R is as defined,

in the presence of an inert gas, in the presence of the corresponding alcohol and in the presence or absence of NO, on a supported platinum metal catalyst, preferably on a supported palladium catalyst, particularly preferably on a supported Pd(II) halide catalyst and very particularly preferably on a supported $PdCl_2$-containing catalyst, at elevated temperature in a continuous gas phase reaction, characterized in that the reaction is carried out at a volume ratio of alkyl nitrite:CO = 0.1 to 10:1, preferably 0.2 to 4:1 and particularly preferably 0.3 to 3:1, a pressure of 0.5 to 10 bar, preferably 0.8 to 7 bar, particularly preferably 1 to 6 bar and very particularly preferably 1 to 5 bar, and a temperature of 50 to 170°C, preferably 70 to 160°C and particularly preferably 70 to 150°C, halogen being added batchwise or continuously.

2. Process according to Claim 1, characterized in that halogen is added in an amount such that the educt gas contains the halogen in a concentration of 1 to 1000 ppm (by volume), preferably 10 - 500 ppm (by volume).

3. Process according to Claim 1, characterized in that chlorine or bromine, preferably chlorine, is added.

## Revendications

1. Procédé de préparation de carbonates de dialkyle de formule :

$$O=C(OR)_2$$

dans laquelle
R représente un groupement alkyle en $C_1$-$C_4$ à chaîne linéaire ou ramifiée, de préférence le méthyle ou l'éthyle, mieux le méthyle,
par réaction du monoxyde de carbone (CO) avec des nitrites d'alkyle de formule :

RONO

dans laquelle
R est tel que défini ci-dessus,
en présence d'un gaz inerte ainsi qu'en présence de l'alcool de base, ainsi qu'en présence ou en l'absence de NO, sur un catalyseur support à base de métal platine, de préférence sur un catalyseur support à base de palladium, mieux sur un catalyseur support à base d'halogénure de Pd(II), encore mieux sur un catalyseur support contenant du $PdCl_2$, à une température élevée dans une réaction en phase gazeuse continue,
caractérisé en ce qu'on opère à un rapport de volume du nitrite d'alkyle au CO de 0,1 à 10:1, de préférence de 0,2 à 4:1 et mieux de 0,3 à 3:1, à une pression de 0,5 à 10 bar, de préférence de 0,8 à 7 bar, mieux de 1 à 6 bar et encore mieux de 1 à 5 bar, et à une température de 50 à 170°C, de préférence de 70 à 160°C et mieux de 70 à 150°C, étant entendu qu'on ajoute un halogène en discontinu ou en continu.

2. Procédé selon la revendication 1, caractérisé en ce qu'on ajoute l'halogène en une quantité telle que le gaz éduit contient l'halogène en une concentration de 1 à 1000 ppm (volume) et de préférence de 10 à 500 ppm (volume).

3. Procédé selon la revendication 1, caractérisé en ce qu'on ajoute du chlore ou du brome et de préférence du chlore.